# EUROPÄISCHE PATENTSCHRIFT

(11) **EP 0 477 141 B1**
(45) Veröffentlichungstag und Bekanntmachung des Hinweises auf die Patenterteilung: **26.02.1997**
(21) Anmeldenummer: 91810725.1
(22) Anmeldetag: 11.09.1991
(51) Int. Cl.: C07D 275/02, C07D 277/22, C07D 261/08, C07D 263/10, C07D 285/06, C07D 285/08, C07D 285/10, C07D 285/125, C07D 271/04, C07D 271/06, C07D 271/08, A61K 31/41

(54) **Heteroarylmethylbenzole-Aromatasehemmer**
Heteroarylmethylbenzols aromatase inhibitors
Hétéroarylméthylbenzènes inhibiteurs de l'aromatase

(30) Priorität: 18.09.1990 CH 3014/90
(43) Veröffentlichungstag der Anmeldung: 25.03.1992
(73) Patentinhaber: CIBA-GEIGY AG, 4002 Basel (CH)
(72) Erfinder: Lang, Marc, Dr., F-68200 Mulhouse (FR)

(56) Entgegenhaltungen:
- EP-A- 0 165 784

## Beschreibung

Die Erfindung betrifft Verbindungen der Formel I, 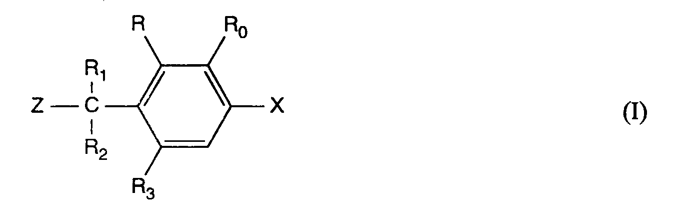 worin Z einen fünfgliedrigen, Stickstoff enthaltenden heteroaromatischen Ring ausgewählt aus der Gruppe 5-Isothiazolyl, 5-Thiazolyl, 5-Isoxazolyl, 5-Oxazolyl, 5-(1,2,3-Thiadiazolyl), 5-(1,2,3-Oxadiazolyl), 3-(1,2,5-Thiadiazolyl), 3-(1,2,5-Oxadiazolyl), 4-Isothiazolyl, 4-Isoxazolyl, 4-(1,2,3-Thiadiazolyl), 4-(1,2,3-Oxadiazolyl), 2-(1,3,4-Thiadiazolyl), 2-(1,3,4-Oxadiazolyl), 5-(1,2,4-Thiadiazolyl) und 5-(1,2,4-Oxadiazolyl) bedeutet; R, R₀ und R₃ Wasserstoff bedeuten; R₁ Wasserstoff, Methyl, Hydroxy oder Fluor bedeutet; R₂ für Cyanophenyl steht; oder R₁ und R₂ zusammen Methyliden bedeuten; oder R₂ und R₃ zusammen -(CH₂)₃- bedeuten; oder R₁ und R₂ und R₃ zusammen für eine Gruppe =CH-(CH₂)₂- stehen, wobei die Einfachbindung am Benzolring angeknüpft ist; und X für Cyano steht; und Salze davon, Verfahren zur Herstellung dieser Verbindungen, pharmazeutische Präparate, die diese Verbindungen enthalten, und die Verwendung dieser Verbindungen zur Herstellung pharmazeutischer Präparate zur therapeutischen Behandlung des menschlichen oder tierischen Körpers.

Die Verbindungen der Formel I, welche ein asymmetrisches Kohlenstoffatom enthalten, können jeweils als Racemat oder als R- oder S-Enantiomer vorliegen. Die Erfindung bezieht sich auf alle diese Formen sowie z.B. auch auf Diastereomere und Gemische davon, die auftreten können, wenn zwei oder mehr asymmetrische Zentren im Molekül vorhanden sind, sowie auf geometrische Isomere, z.B. cis- und trans-Isomere, wenn das Molekül eine Doppelbindung enthält.

Die europäische Patentanmeldung EP 0 165 784 nennt Aromataseinhibitoren, die sich in mindestens 2 Strukturmerkmalen von den Verbindungen der Formel I unterscheiden.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7, insbesondere bis und mit 4, und vor allen Dingen 1 oder 2 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Bedeuten R₂ und R₃ zusammen -(CH₂)₃-, so bilden diese Reste zusammen mit dem zentralen Kohlenstoffatom und dem Benzolring ein Tetralingerüst.

Bedeuten R₁ und R₂ und R₃ zusammen eine Gruppe =CH-(CH₂)₂-, wobei die Einfachbindung jeweils am Benzolring angeknüpft ist, so bilden diese Reste zusammen mit dem zentralen Kohlenstoffatom und dem Benzolring ein 1,2-Dihydronaphthalingerüst. Letzteres kann auch - wie in den Beispielen geschehen - als 3,4-Dihydronaphthalin bezeichnet werden.

Halogen steht insbesondere für Chlor und Brom, kann aber auch Fluor oder Iod bedeuten.

Carbamoyl steht für die Gruppe -CONH₂.

Aryl allgemein bedeutet bevorzugt Phenyl, das unsubstituiert oder durch Niederalkyl, Niederalkoxy, Hydroxy, Halogen oder Trifluormethyl substituiert ist, und vor allen Dingen Phenyl.

Salze von erfindungsgemässen Verbindungen sind in erster Linie pharmazeutisch verwendbare, nicht-toxische Salze. Beispielsweise können Verbindungen der Formel I mit basischen Gruppen Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder mit Aminosäuren, wie Arginin oder Lysin. Verbindungen der Formel I mit einer sauren Gruppe, z.B. Carboxy, bilden z.B. Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)amin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylaminen, z.B. N,N'-Dibenzylethylendiamin, Dibenzylamin oder Benzyl-ß-phenethylamin. Verbindungen der Formel I mit einer sauren und einer basischen Gruppe können auch in Form von inneren Salzen, d.h. in zwitterionischer Form, vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Die erfindungsgemässen Verbindungen der Formel I weisen wertvolle, insbesondere pharmakologische, Eigenschaften auf. Insbesondere hemmen sie selektiv das Enzym Aromatase bei Säugern einschliesslich Menschen. Dadurch wird die metabolische Umwandlung von Androgenen zu Oestrogenen gehemmt. Die Verbindungen der Formel I sind daher z.B. zur Behandlung Oestrogen-abhängiger Krankheiten, einschliesslich Oestrogen-abhängigem Brustkrebs, insbesondere bei postmenopausalen Frauen, geeignet. Ferner sind sie z.B. nützlich bei der Behandlung von Gynäkomastie, d.h. männlicher Brustentwicklung, weil die Aromatisierung der Steroide gehemmt wird.

Diese Wirkungen können durch in vitro-Versuche oder in vivo-Versuche, vorzugsweise an Säugetieren, z.B. Meerschweinchen, Mäusen, Ratten, Katzen, Hunden oder Affen, nachgewiesen werden. Die angewandte Dosierung liegt z.B. in einem Bereich von etwa 0,001 und 10 mg/kg, vorzugsweise zwischen 0,001 und 1 mg/kg.

Die in vitro-Hemmung der Aromataseaktivität kann z.B. mit der Methode, die in J. Biol. Chem. 249, 5364 (1974) beschrieben ist, gezeigt werden. Ferner können IC₅₀-Werte für die Aromatasehemmung z.B. in vitro aus enzymkinetischen Studien, die die Hemmung der Umwandlung von 4-¹⁴C-Androstendion zu 4-¹⁴C-Oestron in menschlichen plazentalen Mikrosomen betreffen, erhalten werden. Die IC₅₀-Werte der erfindungsgemässen Verbindungen liegen minimal etwa bei 10⁻⁹ M.

In vivo kann die Aromatasehemmung z.B. durch die Unterdrückung des ovarialen Oestrogengehalts weiblicher Ratten gezeigt werden, die zunächst mit Stutenserumgonadotropin und 2 Tage später mit menschlichem Choriongonadotropin injiziert werden, am folgenden Tag p.o. mit einer Verbindung der Erfindung behandelt werden und 1 h später mit Androstendion. Eine weitere Möglichkeit zur in vivo Bestimmung der Aromatasehemmung wird im folgenden beschrieben: Androstendion (30 mg/kg subkutan) wird allein bzw. zusammen mit einer Verbindung der Erfindung (oral oder subkutan) 4 Tage lang an nicht geschlechtsreife weibliche Ratten verabreicht. Nach der vierten Anwendung werden die Ratten getötet, die Uteri isoliert und gewogen. Die Aromatasehemmung wird bestimmt durch das Ausmass, in dem die durch die Verabreichung von Androstendion allein verursachte Uterushypertrophie durch die gleichzeitige Verabreichung der erfindungsgemässen Verbindung unterdrückt bzw. verringert wird. Die minimale effektive Dosis der erfindungsgemässen Verbindungen bei den in vivo Tests liegt etwa zwischen 0,001 und 1 mg/kg.

Die Antitumorwirkung, insbesondere bei Oestrogen-abhängigen Tumoren, kann in vivo z.B. bei DMBA-induzierten Mammatumoren an weiblichen Sprague-Dawley-Ratten gezeigt werden [vgl. Proc. Soc. Exp. Biol. Med. 160, 296-301 (1979)]. Die Anwendung von erfindungsgemässen Verbindungen bewirkt eine Regression der Tumoren und unterdrückt weiterhin das Auftreten neuer Tumoren bei täglichen Dosen ab etwa 1 mg/kg p.o.

Darüberhinaus haben die Verbindungen der Formel I keine Hemmwirkung auf die Spaltung der Cholesterin-Seitenkette und induzieren keine adrenale Hypertrophie, was durch endokrine Organuntersuchungen gezeigt wird.

Aufgrund ihrer pharmakologischen Eigenschaften als äusserst selektive Inhibitoren des Enzyms Aromatase sind die Verbindungen der Formel I z.B. zur Behandlung Oestrogen-abhängiger Krankheiten, wie Brusttumoren (Brustkarzinoma), Endometriosis, vorzeitigen Wehen oder endometrialen Tumoren bei Frauen oder Gynäkomastie bei Männern, geeignet.

Vor allen Dingen betrifft die Erfindung die Verbindungen der Formel I, worin Z 5-Isothiazolyl oder 5-Thiazolyl bedeutet; R, R₀ und R₃ Wasserstoff bedeuten; R₁ Wasserstoff, Methyl, Hydroxy oder Fluor bedeutet; R₂ für Cyanophenyl steht; oder R₁ und R₂ zusammen Methyliden bedeuten; oder R₂ und R₃ zusammen -(CH₂)₃- bedeuten; oder R₁ und R₂ und R₃ zusammen für eine Gruppe =CH-(CH₂)₂- stehen, wobei die Einfachbindung am Benzolring angeknüpft ist; und X für Cyano steht; und Salze davon.

Insbesondere betrifft die Erfindung die Verbindungen der Formel I, worin Z 5-Isothiazolyl oder 5-Thiazolyl bedeutet; R, R₀ und R₃ Wasserstoff bedeuten; R₁ Wasserstoff, Hydroxy oder Fluor bedeutet; R₂ für Cyanophenyl steht; oder R₁ und R₂ zusammen Methyliden bedeuten; oder R₂ und R₃ zusammen -(CH₂)₃- bedeuten; oder R₁ und R₂ und R₃ zusammen für eine Gruppe =CH-(CH₂)₂- stehen, wobei die Einfachbindung am Benzolring angeknüpft ist; und X für Cyano steht; und Salze davon.

Als Untergruppen aus einer Gruppe von Verbindungen der Formel I sind jeweils hervorzuheben: (a) Verbindungen der Formel I, worin R₁ Wasserstoff, Hydroxy oder Fluor bedeutet; (b) Verbindungen der Formel I, worin R₁ Wasserstoff bedeutet; (c) Verbindungen der Formel I, worin Z 5-Isothiazolyl bedeutet; und (d) Verbindungen der Formel I, worin Z 5-Thiazolyl bedeutet.

Die Erfindung betrifft vor allem die in den Beispielen beschriebenen spezifischen Verbindungen und pharmazeutisch verwendbare Salze davon.

Die Verbindungen der Formel I können in an sich bekannter Weise hergestellt werden, indem man z.B.
(a) zur Herstellung einer Verbindung der Formel I, worin R₁ Hydroxy bedeutet, eine Verbindung der Formel II, 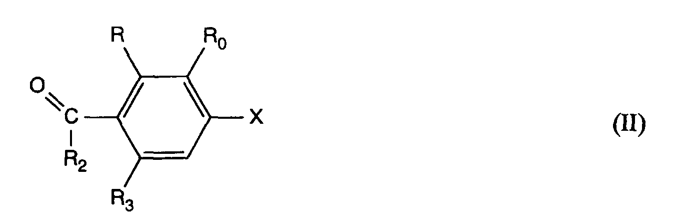 worin X, R, R₀, R₂ und R₃ wie unter Formel I definiert sind, mit einer Verbindung der Formel III,

   Z-R₄ (III)

   worin R₄ für Wasserstoff oder eine an einem Ringkohlenstoff sitzende Schutzgruppe oder Abgangsgruppe steht und Z wie unter Formel I definiert ist, umsetzt, oder
(b) zur Herstellung einer Verbindung der Formel I, worin R₁ und R₂ zusammen Methyliden bedeuten, eine Verbindung der Formel IV, 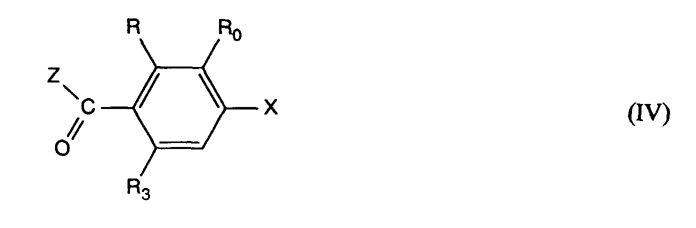 worin Z, R, R₀, R₃ und X wie unter Formel I definiert sind, mit einer Verbindung der Formel V,

   Alk = W₁ (V)

   worin Alk Methyliden und W₁ eine Phosphoranylidengruppe bedeutet, umsetzt, oder
(c) in einer Verbindung der Formel VI, 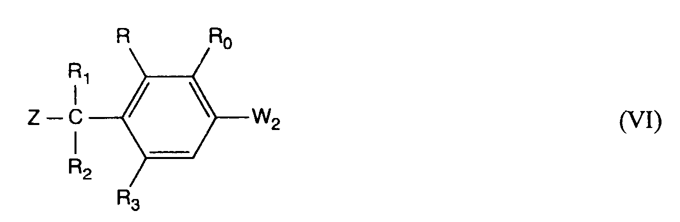 worin Z, R, R₀, R₁, R₂ und R₃ wie unter Formel I definiert sind und W₂ eine in den Rest X überführbare Gruppe bedeutet, W₂ in den Rest X überführt, wobei gegebenenfalls ein mit W₂ identischer Substituent innerhalb der Gruppe R₂ gleichzeitig umgewandelt wird, oder
(d) zur Herstellung einer Verbindung der Formel I, worin R₁ Wasserstoff bedeutet, eine Verbindung der Formel I, worin R₁ Hydroxy bedeutet, reduziert, oder
(e) zur Herstellung einer Verbindung der Formel I, worin R₁ und R₂ und R₃ zusammen für eine Gruppe =CH-(CH₂)₂- stehen, aus einer Verbindung der Formel I, worin R₂ und R₃ zusammen -(CH₂)₃- bedeuten und R₁ für Hydroxy steht, Wasser eliminiert, oder
(f) zur Herstellung einer Verbindung der Formel I, worin R₁ Methyl bedeutet, eine Verbindung der Formel I, worin R₁ Wasserstoff bedeutet, mit Methanol, oder einem reaktiven funktionellen Derivat davon, kondensiert, und/oder
eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I in ein Salz umwandelt, und/oder ein erhaltenes Gemisch von isomeren Verbindungen der Formel I in die einzelnen Isomeren auftrennt.

In der folgenden Beschreibung der Verfahren (a), (b), (c), (d) und (e) haben die Symbole Z, R, R₀, R₁, R₂, R₃ und X jeweils die unter Formel I angegebene Bedeutung, sofern nichts anderes angegeben ist.

Verfahren (a): In einer Verbindung der Formel III bedeutet eine an einem Ringkohlenstoff sitzende Schutzgruppe oder Abgangsgruppe R₄ z.B. Triniederalkylsilyl. Als Schutzgruppe fungiert Triniederalkylsilyl z.B. im 2-Trimethylsilylthiazol, wo die 2-Stellung des Thiazols blockiert wird. Als Abgangsgruppe fungiert Triniederalkylsilyl z.B im 5-Trimethylsilylthiazol, wo die Verknüpfung gemäss Verfahren (a) in Gegenwart von z.B. Caesiumfluorid in 5-Stellung des Thiazols erfolgt. Eine weitere universell verwendbare Abgangsgruppe ist z.B. Halogen.

Bei der Umsetzung gemäss dem Verfahren (a) wird normalerweise eine Verbindung der Formel III, worin R₄ Wasserstoff oder eine Abgangsgruppe bedeutet, mit einer - bevorzugt starken - Base, z.B. einer Alkalimetallbase, etwa n-Butyllithium, Natriumhydrid oder Lithiumdiisopropylamid (LDA), zur Reaktion gebracht und dann mit dem Keton bzw. Aldehyd der Formel II umgesetzt.

In einer Variante des Verfahrens (a) wird der durch eine Abgangsgruppe R₄ in der beabsichtigten Verknüpfungsposition substituierte Heteroaromat Z-R₄ in Gegenwart eines Aktivators, z.B. Caesiumfluorid, mit dem Aldehyd bzw. Keton der Formel II umgesetzt. Bei dieser Reaktion erhält man, wenn ein Aldehyd der Formel II eingesetzt wird, manchmal neben der gewünschten Verbindung der Formel I mit R₁ = OH auch den entsprechenden acylierten Heteroaromaten als Nebenprodukt. Letzterer entsteht vermutlich durch Oxidation des zunächst gebildeten Alkohols der Formel I.

Die Ausgangsverbindungen der Formel II und III sind an sich bekannt oder können analog zu bekannten Verbindungen hergestellt werden.

Verfahren (b): In einer Verbindung der Formel V bedeutet eine Phosphoranylidengruppe W₁ einen Rest = P(W₃, W₄, W₅), worin W₃, W₄ und W₅ gleich oder verschieden sein können und z.B. Niederalkoxy, Niederalkyl oder Aryl bedeuten. Beispielhaft seien Triphenylphosphin oder Diethylphosphono genannt.

Das Verfahren (b) entspricht der Wittig-Reaktion und Modifikationen davon und ist an sich bekannt. Die Ausgangsverbindungen der Formel V werden z.B. aus den entsprechenden Phosphoniumsalzen der Formel Methyl-P(W₃, W₄, W₅)^{⊕}A^{⊖}, worin A^{⊖} ein Anion, z.B. Chlorid oder Bromid, bedeutet, durch Behandlung mit einer Base, z.B. n-Butyllithium, enthalten.

Verfahren (c): In einer Verbindung der Formel VI steht eine in den Rest X überführbare Gruppe W₂ z.B. für Halogen, Amino, Carboxy, Niederalkoxycarbonyl, Halogencarbonyl, ein Säureanhydrid, Hydroxy, Niederalkoxy, Arylniederalkoxy oder Aryloxy.

W₂ = Halogen, insbesondere Brom, kann z.B. durch Umsetzung mit einem Cyanierungsmittel, z.B. Kupfer(I)cyanid, in Cyano überführt werden. W₂ = Amino kann z.B. via Diazotierung beispielsweise in Halogen, Cyano oder Hydroxy umgewandelt werden.

Die Umwandlung von Substituenten an aromatischen Systemen gemäss Verfahren (c) ist an sich bekannt.

Für den Fall, dass in einer Verbindung der Formel VI die Gruppe R₂ einen mit W₂ identischen Substituenten enthält, z.B. bei R₂ = -C₆H₄-W₂, kann dieser bei der Durchführung des Verfahrens (c) gleichzeitig mit umgewandelt werden.

Die Ausgangsverbindungen der Formel VI werden z.B. analog Verfahren (a) bis (f) hergestellt, wobei in den entsprechenden Umsetzungen anstelle des Restes X ein Rest W₂ eingesetzt wird.

Die Verbindung der Formel VI namens Bis-(4,4'-bromphenyl)-(5-isothiazolyl)-methan, oder ein pharmazeutisch verwendbares Salz davon, weist ihrerseits wertvolle pharmakologisch verwendbare Eigenschaften auf und bildet daher einen weiteren Gegenstand der Erfindung. Sie wirkt in gleicher Weise wie die Verbindungen der Formel I als Aromatasehemmer. Ihre Aktivität liegt in der gleichen Grössenordnung wie die der Verbindungen der Formel I (s. oben). Daher ist auch die genannte Verbindung der Formel VI nützlich z.B. zur Behandlung der oben bei den Verbindungen der Formel I angegebenen Krankheiten.

Die Erfindung betrifft daher auch diese neue Verbindung der Formel VI, zum einen als Zwischenprodukt zur Herstellung der wertvollen Verbindungen der Formel I und zum anderen als pharmazeutischen Wirkstoff, sowie pharmazeutische Präparate enthaltend diese Verbindung der Formel VI zusammen mit einem oder mehreren pharmazeutisch verwendbaren Trägermaterialien. Die Erfindung betrifft ferner auch die Verwendung der genannten Verbindung der Formel VI zur Herstellung von pharmazeutischen Präparaten für die Tumorbehandlung.

Verbindungen der Formel I können in andere Verbindungen der Formel I umgewandelt werden.

Zum Beispiel können gemäss Verfahren (d) Verbindungen der Formel I, worin R₁ Hydroxy bedeutet, durch Reduktion, z.B. mit Zinn(II)chlorid oder Eisessig/wässrige Iodwasserstoffsäure, in Verbindungen der Formel I, worin R₁ Wasserstoff bedeutet, überführt werden.

Umgekehrt können auch Verbindungen der Formel I, worin R₁ Wasserstoff bedeutet, durch Oxidation, z.B. mit Sauerstoff im basischen Medium, in Verbindungen der Formel I, worin R₁ Hydroxy bedeutet, überführt werden.

Zur Einführung von R₁ = Fluor ist insbesondere die folgende Methode geeignet: Aus der entsprechenden Verbindung der Formel I mit R₁ = H wird durch Behandlung mit einer starken Base, z.B. einem Alkalimetall-diisopropylamid, etwa Lithium-, Natrium- oder Kaliumdiisopropylamid, oder einer Hexamethyldisilazidbase, etwa Kalium-, Natrium- oder Lithiumhexamethyldisilazid, oder einer Alkalimetallbase, etwa sek.-, tert.- oder n-Butyllithium, das entsprechende Carbanion gebildet und letzteres mit einem elektrophilen Fluorierungsmittel, z.B. einem N-F-Sulfonamid [s. z.B. J. Amer. Chem. Soc. 106, 452 (1984) oder J. Fluor. Chem. 46, 297 (1990)], insbesondere N-Fluor-3,3-dimethyl-2,3-dihydro-1,2-benzothiazol-1,1-dioxid [s. Helv. Chim. Acta 72, 1248 (1989) und Chimia 44, 120 (1990)], umgesetzt.

Weitere elektrophile Fluorierungsmittel, die in Frage kommen, sind zum Beispiel: N-F-Sulfonimide [s. z.B. J. Amer. Chem. Soc. 109, 7194 (1987)], N-F-Pyridiniumderivate [s. z.B. J. Amer. Chem. Soc. 112, 8563 (1990)], insbesondere N-Fluor-2,4,6-trimethylpyridinium-trifluormethylsulfonat, N-F-Perfluorpiperidine [s. z.B. Chem. and Industry (London) 1964, 1864], N-F-Quinuclidiniumsalze [s. z.B. J. Fluor. Chem. 41, 297 (1988) oder J. Chem. Soc. Perkin Trans. I 1988, 2805], N-F-Pyridone [s. z.B. J. Fluor. Chem. 26, 43 (1984)] oder N-F-Amide oder -Lactame [s. z.B. J. Org. Chem. 55, 3373 (1990)].

Des weiteren können Verbindungen der Formel I, worin R₁ Hydroxy bedeutet, auch durch Umsetzung mit bestimmten Fluorierungsmitteln, etwa sekundären Amino-schwefeltrifluoriden, z.B. Piperidino-schwefeltrifluorid oder Diethylamino-schwefeltrifluorid ["DAST", s. z.B. J. Org. Chem. 40, 574 (1975)], oder SF₄ (s. z.B. Organic Reactions 34, 319, Wiley, New York etc. 1985), z.B. im System SF₄/HF, in Verbindungen der Formel I, worin R₁ Fluor bedeutet, überführt werden.

Weiter können gemäss Verfahren (e) Verbindungen der Formel I, worin R₂ und R₃ zusammen -(CH₂)₃- bedeuten und R₁ für Hydroxy steht, durch Umsetzung mit einem Wasser eliminierenden Mittel, z.B. Thionylchlorid, in Verbindungen der Formel I, worin R₁ und R₂ und R₃ zusammen für eine Gruppe =CH-(CH₂)₂- stehen, überführt werden.

Weiterhin können gemäss Verfahren (f) Verbindungen der Formel I, worin R₁ Wasserstoff bedeutet, durch Umsetzung mit Methanol, oder insbesondere einem reaktiven funktionellen Derivat davon, in Verbindungen der Formel I, worin R₁ Methyl bedeutet, überführt werden. Dabei werden die Ausgangsverbindungen (R₁ = H) zunächst mit einer starken Base, z.B. einer Hexamethyldisilazidbase, etwa Natrium-, Lithium- oder vor allem Kaliumhexamethyldisilazid, oder einem Alkalimetall-niederalkoxid, z.B. Kalium-tert.-butoxid, in das entsprechende Carbanion überführt und dann insbesondere mit einem Methylhalogenid, in erster Linie mit Methyliodid, alkyliert.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -80°C bis etwa 200°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen.

Die Verbindungen, einschliesslich ihrer Salze, können auch in Form von Hydraten erhalten werden, oder ihre Kristalle können z.B. das zur Kristallisation verwendete Lösungsmittel einschliessen.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 0,1 % bis etwa 95 % des Wirkstoffs, wobei einzeldosierte Applikationsformen vorzugsweise von etwa 1 % bis etwa 90 % und nicht-einzeldosierte Applikationsformen vorzugsweise etwa 0,1 % bis etwa 20 % Wirkstoff aufweisen. Dosiseinheitsformen, wie Dragees, Tabletten oder Kapseln, enthalten von etwa 0,5 mg bis etwa 100 mg des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragee-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder tlüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 0,01 % bis 2 %, vorzugsweise ca. 0,1 *%* oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,5 mg bis etwa 100 mg, vorzugsweise von etwa 1 mg bis etwa 20 mg, einer Verbindung der vorliegenden Erfindung verabreicht.

Die nachfolgenden Beispiele illustrieren die vorliegende Erfindung; Temperaturen werden in Grad Celsius angegeben. Folgende Abkürzungen werden verwendet: Ether = Diethylether; Ethylacetat = Essigsäureethylester; THF = Tetrahydrofuran; Hexan = n-Hexan; DMSO = Dimethylsulfoxid; DMF = Dimethylformamid; DC = Dünnschichtchromatographie; RT = Raumtemperatur.

### Beispiel 1: 4-[α-(4-Cyanophenyl)-α-hydroxy-5-isothiazolylmethyl]-benzonitril

0,41 ml Diisopropylamin werden in 5 ml abs. THF gelöst, auf -75° gekühlt und mit 1,53 ml 1,6 M n-Butyllithium in Hexan versetzt. Diese Lösung wird tropfenweise zu einem auf -75° gekühlten, gerührten Gemisch von 204 mg Isothiazol in 18 ml THF gegeben. Nach 1 h Rühren bei -75° wird das Gemisch mit einer Lösung von 539 mg 4,4'-Dicyanobenzophenon in 5 ml THF versetzt. Nach weiteren 10 min Rühren wird das Kühlbad entfernt, und nach Erreichen einer Temperatur von 0° wird das Reaktionsgemisch auf 40 ml einer eisgekühlten, gesättigten wässrigen Ammoniumchlorid-Lösung gegossen. Das Produkt wird mehrmals mit Ether extrahiert, und die vereinten organischen Extrakte werden nach Trocknen über Natriumsulfat eingeengt. Das Rohprodukt wird durch Säulenchromatographie gereinigt (SiO₂, Hexan/Ethylacetat 2:1) und aus Hexan kristallisiert; Smp. 162-164°; ¹H-NMR (CDCl₃): δ (ppm) = 3,37 (s,1H), 7,0 (d,1H), 7,5 und 7,7 (m,8H), 8,46 (d,1H).

Das Ausgangsmaterial wird wie folgt hergestellt:

### (a) 4,4'-Dicyanobenzophenon

Eine Lösung von 5,1 g 4,4'-Dibrombenzophenon in 90 ml DMF wird mit 8,13 g CuCN versetzt und während 13 h unter Rückfluss gerührt. Nach dem Abkühlen wird das Reaktionsgemisch mit Ethylacetat verdünnt, zweimal mit 50 % wässriger Ethylendiamin-Lösung, zweimal mit Wasser und noch dreimal mit Sole gewaschen, getrocknet und eingeengt. Nach Säulenchromatographie (SiO₂, Toluol bis Toluol/Ethylacetat 95:5) erhält man die kristalline Titelverbindung; DC (Toluol/Ethylacetat 9:1): Rf = 0,34; IR (CH₂Cl₂): 2220, 1670, 1605, 1405 cm⁻¹.

### Beispiel 2: 4-[α-(4-Cyanophenyl)-5-isothiazolylmethyl]-benzonitril

Analog Beispiel 4 werden 208 mg 4-[α-(4-Cyanophenyl)-α-hydroxy-5-isothiazolylmethyl]-benzonitril (Beispiel 1) in 2,38 ml Eisessig mit 296 mg Zinn(II)chlorid und 0,761 ml 36,5 % Salzsäure in die Titelverbindung überführt. Die Reinigung wird mittels Säulenchromatographie (SiO₂, 0,2 bar, Hexan/Ethylacetat 3:1) und Kristallisation aus Hexan durchgeführt; Smp. 95-98°; ¹H-NMR (CDCl₃): δ (ppm) = 5,9 (s,1H), 6,95 (d,1H), 7,32 und 7,7 (m,8H), 8,48 (d,1H); IR (CH₂Cl₂): 2220, 1600 cm⁻¹.

### Beispiel 3: 4-[α-(4-Cyanophenyl)-α-hydroxy-5-thiazolylmethyl]-benzonitril

Analog Beispiel 1 wird ausgehend von 0,512 ml Diisopropylamin, 2,44 ml einer 1,6 M n-Butyllithium-Lösung, 530 mg 2-Trimethylsilylthiazol und 603 mg 4,4'-Dicyanobenzophenon in THF bei -70° und gleicher Aufarbeitung die Titelverbindung hergestellt. Nach Reinigung durch Säulenchromatographie (SiO₂, Hexan/Ethylacetat 2:1) erhält man die Titelverbindung in reiner Form. DC (Hexan/Ethylacetat 2:1) Rf = 0,2; ¹H-NMR (CDCl₃): δ (ppm) = 4,39 (s,1H), 7,42 (d,1H), 7,54 und 7,7 (m,8H), 7,88 (d,1H).

### Beispiel 4: 4-[α-(4-Cyanophenyl)-5-thiazolylmethyl]-benzonitril

235 mg 4-[α-(4-Cyanophenyl)-α-hydroxy-5-thiazolylmethyl]-benzonitril (Beispiel 3) werden in 2,68 ml Eisessig gelöst und nacheinander mit 334 mg Zinn(II)chlorid und 0,86 ml 36,5 % Salzsäure versetzt. Die gelbe Lösung wird 2 h bei 120° und dann über Nacht bei RT gerührt. Das farblose Reaktionsgemisch wird mit 9,2 ml Wasser verdünnt, der auskristallisierende Feststoff abgenutscht und bei 50° unter vermindertem Druck getrocknet. Dann wird er in wenig THF gelöst und mit 0,059 ml Pyridin verrührt. Nach Eindampfen wird der Rückstand in Ethylacetat aufgenommen, nacheinander mit Wasser, 2N HCl, Wasser und Sole gewaschen und nach dem Trocknen über Natriumsulfat eingeengt. Eine Säulenchromatographie (SiO₂, Hexan/Ethylacetat 1:1) liefert die Titelverbindung, die aus Ether kristallisiert wird; Smp. 54-56°; ¹H-NMR (CDCl₃): δ (ppm) = 4,31 (s,1H), 7,42 (d,1H), 7,53 und 7,68 (m,8H), 7,88 (d,1H).

### Beispiel 5: Bis-(4,4'-bromphenyl)-(5-isothiazolyl)-methan

Analog Beispiel 4 werden 170 mg Bis-(4,4'-bromphenyl)-(5-isothiazolyl)-methanol in 1,45 ml Eisessig mit 180 mg Zinn(II)chlorid und 0,464 ml 36,5 % Salzsäure in die Titelverbindung überführt. Nach gleicher Aufarbeitung und Reinigung durch Säulenchromatographie (SiO₂, Hexan bis Hexan/Ethylacetat 20:1) und mehrmaligem Kristallisieren aus Hexan erhält man die reine Titelverbindung; Smp. 63-67°; ¹H-NMR (CDCl₃): δ (ppm) = 5,6 (s,1H), 6,92 (d,1H), 7,07 und 7,48 (m,8H), 8,41 (d,1H).

Das Ausgangsmaterial wird wie folgt hergestellt:

### Beispiel 5 (a): Bis-(4,4'-bromphenyl)-(5-isothiazolyl)-methanol

Analog Beispiel 1 wird ausgehend von 0,34 ml Diisopropylamin, 1,25 ml einer 1,6 M n-Butyllithium-Lösung, 170 mg Isothiazol und 680 mg 4,4'-Dibrombenzophenon in THF bei -75° und gleicher Aufarbeitung die Titelverbindung hergestellt. Diese wird durch Säulenchromatographie (SiO₂ 0,04-0,06 mesh, 0,2 bar, Hexan/Ethylacetat 4:1) gereinigt und aus Hexan kristallisiert; Smp. 139-140°; ¹H-NMR (CDCl₃): δ (ppm) = 3,1 (s,1H), 6,99 (d,1H), 7,22 und 7,49 (m,8H), 8,41 (d,1H).

### Beispiel 6: 1-(4-Cyanophenyl)-1-(5-thiazolyl)-ethylen

714 mg Methyltriphenylphosphoniumbromid werden in 6,7 ml Ether vorgelegt und bei 7° mit 0,973 ml 1,6 M n-Butyllithium in Hexan versetzt. Die orange Suspension wird 1,5 h bei der gleichen Temperatur gerührt und dann mit einer auf 7° gekühlten Suspension von 5-(4-Cyanobenzoyl)-thiazol in 3 ml Ether umgesetzt. Nach 3 h Rühren bei RT wird der Ether durch Ethylacetat ersetzt und weitere 2 h bei Rückfluss gerührt. Der Feststoff wird abgenutscht und die Mutterlauge eingeengt. Der Rückstand wird in Methylenchlorid aufgenommen und die organische Phase nacheinander mit 7 ml 2 N Salzsäure und Wasser gewaschen, über Natriumsulfat getrocknet und eingeengt. Das Rohprodukt wird durch Säulenchromatographie (SiO₂ 0,04-0,06 mesh, 0,1 bar, Hexan/Ethylacetat 2:1 bis 1:1) gereinigt und aus Hexan kristallisiert; Smp. 71-73°; ¹H-NMR (CDCl₃): δ (ppm) = 5,5 (s,1H), 5,73 (s,1H) 7,54 und 7,71 (m,4H), 7,69 (s,1H), 8,78 (s,1H).

Das Ausgangsmaterial wird wie folgt hergestellt:

### Beispiel 6 (a): (i) 5-(4-Cyanobenzoyl)-thiazol und (ii) 4-(α-Hydroxy-5-thiazolylmethyl)-benzonitril

Eine Lösung von 9,4 g 5-Trimethylsilylthiazol in 0,56 l THF wird mit 15,7 g 4-Cyanobenzaldehyd und 9,1 g Caesiumfluorid versetzt und 16 h am Rückfluss erhitzt. Der Feststoff wird abfiltriert und das Lösungsmittel eingeengt. Die Säulenchromatographie (SiO₂, Hexan/Ethylacetat 1:2) liefert zuerst (i) das 5-(4-Cyanobenzoyl)-thiazol [Rf Hexan/Ethylacetat 1:2) = 0,65], Smp. (nach Kristallisation aus Ethylacetat) 181-182°; ¹H-NMR (CDCl₃): δ (ppm) = 8,88 und 8 (m,4H), 8,36 (s,1H), 9,14 (s,1H) und anschliessend (ii) das 4-(α-Hydroxy-5-thiazolylmethyl)-benzonitril [Rf (Hexan/Ethylacetat 1:2) = 0,35], Smp. (nach Kristallisation aus Toluol) 115-117°; ¹H-NMR (CDCl₃): δ (ppm) = 3,05 (d,1H), 6,2 (d,1H), 7,58 und 7,7 (m,4H), 7,73 (s,1H), 8,78 (s,1H).

### Beispiel 7: 6-Cyano-1-hydroxy-1-(5-isothiazolyl-1,2,3,4-tetrahydronaphthalin

Analog Beispiel 1 wird ausgehend von 353 mg Isothiazol, 0,632 ml Diisopropylamin, 3 ml 1,6 M n-Butyllithium in Hexan und 546 mg 6-Cyano-1-tetralon in 38 ml THF die rohe Titelverbindung erhalten. Diese wird durch Säulenchromatographie (SiO₂, Hexan/Ethylacetat 4:1) und Kristallisation aus Hexan gereinigt. Smp. 182-184°; ¹H-NMR (d₆-DMSO): δ (ppm) = 1,76 und 1,97 (2m,4H), 2,9 (t,2H), 6,65 (s,1H), 6,93 (d,1H), 7,37 (dd,1H), 7,59 (dd,1H), 7,7 (d,1H), 8,39 (d,1H).

### Beispiel 8: 6-Cyano-1-(5-isothiazolyl)-3,4-dihydronaphthalin

Analog Referenzbeispiel 8 (a) werden 92 mg 6-Cyano-1-hydroxy-1-(5-isothiazolyl)-1,2,3,4-tetrahydronaphthalin (Beispiel 7) durch Umsetzung mit 0,051 ml Thionylchlorid in 0,32 ml Benzol und anschliessend mit 0,112 ml Morpholin in die rohe Titelverbindung überführt. Diese wird durch Säulenchromatographie (SiO₂, 0,1 bar, Hexan/Ethylacetat 4:1) und Kristallisation aus Hexan gereinigt; Smp. 77-81°; ¹H-NMR (CDCl₃): δ (ppm) = 2,5 (m,2H), 2,92 (t,2H), 6,54 (t,2H), 7,2 (d,1H), 7,29 (d,1H), 7,5 (dd,1H), 7,55 (d,1H), 8,5 (d,1H).

### Referenzbeispiel 8 (a):6-Chlor-1-(5-isothiazol)-3,4-dihydronaphthalin

Eine Lösung von 132 mg 6-Chlor-1-hydroxy-1-(5-isothiazolyl)-1,2,3,4-tetrahydronaphthalin in 0,45 ml Benzol wird bei Raumtemperatur mit 0,072 ml Thionylchlorid versetzt und dann 1,5 h bei 75° gerührt. Man engt unter vermindertem Druck ein, versetzt das erhaltene Harz mit 0,156 ml Morpholin und rührt 2,5 h bei 75°. Die schwarze Suspension wird auf 2° gekühlt und mit ebenfalls vorgekühlter 5N HCl behandelt. Danach wird mit festem NaHCO₃ auf pH 8 gestellt und mit Chloroform extrahiert. Die organische Phase wird abgetrennt, über Natriumsulfat getrocknet und eingeengt. Die rohe Titelverbindung wird durch Säulenchromatographie (SiO₂, Hexan/Ethylacetat 4:1) und Kristallisation aus Hexan gereinigt; ¹H-NMR (CDCl₃): δ (ppm) = 2,44 (m,2H), 2,85 (m,2H), 6,37 (t,1H), 7,14 (m,2H), 7,19 (d,1H), 7,21 (s,1H), 8,49 (d,1H).

### Beispiel 9: 6-Cyano-1-hydroxy-1-(5-thiazolyl)-1,2,3,4-tetrahydronaphthalin

Analog Beispiel 1 wird ausgehend von 472 mg 2-Trimethylsilylthiazol, 0,51 ml Diisopropylamin, 1,88 ml 1,6 M n-Butyllithium in Hexan und 513 mg 6-Cyano-1-tetralon in 36 ml THF die rohe Titelverbindung erhalten. Diese wird durch Säulenchromatographie (SiO₂, 0,2 bar, Hexan/Ethylacetat 3:1) und Kristallisation aus Hexan gereinigt. ¹H-NMR (CDCl₃): δ (ppm) = 2,0 (m,2H), 2,25 und 2,42 (2m,2H), 2,97 (t,2H), 3,73 (s,1H), 7,34 (2d,2H), 7,42 (dd,1H), 7,5 (d,1H), 7,75 (d,1H); IR (CH₂Cl₂): 2220 cm⁻¹.

### Beispiel 10: 6-Cyano-1-(5-thiazolyl)-3,4-dihydronaphthalin

Analog Referenzbeispiel 8 (a) werden 192 mg 6-Cyano-1-hydroxy-1-(5-thiazolyl)-1,2,3,4-tetrahydronaphthalin (Beispiel 9) durch Umsetzung mit 0,108 ml Thionylchlorid in 0,67 ml Benzol und anschliessend mit 0,235 ml Morpholin in die rohe Titelverbindung überführt. Diese wird durch Säulenchromatographie, (SiO₂, Hexan/Ethylacetat 9:1) und Kristallisation aus Hexan gereinigt; Smp. 78-79°; ¹H-NMR (CDCl₃): δ (ppm) = 2,51 (m,2H), 2,9 (t,2H), 6,82 (t,1H), 7,34 (d,1H), 7,48 (d,1H), 7,53 (dd,1H), 7,85 (d,1H), 7,92 (d,1H).

### Beispiel 11:

Analog zu den Beispielen 1-10 können auch die folgenden Verbindungen hergestellt werden:
(a) 4-[α-(4-Cyanophenyl)-5-isoxazolylmethyl]-benzonitril
(b) 4-[α-(4-Cyanophenyl)-5-oxazolylmethyl]-benzonitril
(c) 4-[α-(4-Cyanophenyl)-5-(1,2,3-thiadiazolyl)-methyl]-benzonitril
(d) 4-[α-(4-Cyanophenyl)-5-(1,2,3-oxadiazolyl)-methyl]-benzonitril
(e) 4-[α-(4-Cyanophenyl)-3-(1,2,5-thiadiazolyl)-methyl]-benzonitril
(f) 4-[α-(4-Cyanophenyl)-3-(1,2,5-oxadiazolyl)-methyl]-benzonitril
(g) 4-[α-(4-Cyanophenyl)-4-isothiazolylmethyl]-benzonitril
(h) 4-[α-(4-Cyanophenyl)-4-isoxazolylmethyl]-benzonitril
(i) 4-[α-(4-Cyanophenyl)-4-(1,2,3-thiadiazolyl)-methyl]-benzonitril
(j) 4-[α-(4-Cyanophenyl)-4-(1,2,3-oxadiazolyl)-methyl]-benzonitril
(k) 4-[α-(4-Cyanophenyl)-2-(1,3,4-thiadiazolyl)-methyl]-benzonitril
(l) 4-[α-(4-Cyanophenyl)-2-(1,3,4-oxadiazolyl)-methyl]-benzonitril
(m) 4-[α-(4-Cyanophenyl)-5-(1,2,4-thiadiazolyl)-methyl]-benzonitril
(n) 4-[α-(4-Cyanophenyl)-5-(1,2,4-oxadiazolyl)-methyl]-benzonitril
(o) 6-Cyano-1-(5-isoxazolyl)-3,4-dihydronaphthalin
(p) 6-Cyano-1-(5-oxazolyl)-3,4-dihydronaphthalin
(q) 6-Cyano-1-[5-(1,2,3-thiadiazolyl)]-3,4-dihydronaphthalin
(r) 6-Cyano-1-[5-(1,2,3-oxadiazolyl)]-3,4-dihydronaphthalin
(s) 6-Cyano-1-[3-(1,2,5-thiadiazolyl)]-3,4-dihydronaphthalin
(t) 6-Cyano-1-[3-(1,2,5-oxadiazolyl)]-3,4-dihydronaphthalin
(u) 6-Cyano-1-(4-isothiazolyl)-3,4-dihydronaphthalin
(v) 6-Cyano-1-(4-isoxazolyl)-3,4-dihydronaphthalin
(w) 6-Cyano-1-[4-(1,2,3-thiadiazolyl)]-3,4-dihydronaphthalin
(x) 6-Cyano-1-[4-(1,2,3-oxadiazolyl)]-3,4-dihydronaphthalin
(y) 6-Cyano-1-[2-(1,3,4-thiadiazolyl)]-3,4-dihydronaphthalin
(z) 6-Cyano-1-[2-(1,3,4-oxadiazolyl)]-3,4-dihydronaphthalin
(aa) 6-Cyano-1-[5-(1,2,4-thiadiazolyl)]-3,4-dihydronaphthalin
(ab) 6-Cyano-1-[5-(1,2,4-oxadiazolyl)]-3,4-dihydronaphthalin

### Beispiel 12: 4-[α-(4-Cyanophenyl-5-isothiazolylmethy]-benzonitril

Analog Beispiel 1a werden 500 mg Bis-(4,4'-bromphenyl)-(5-isothiazolyl)-methan (Beispiel 5) in DMF mit CuCN in die Titelverbindung überführt, Smp. 95-98°.

### Beispiel 13: 4-[α-(4-Cyanophenyl)-5-thiazolylmethyl]-benzonitril

Analog Beispiel 1a wird Bis-(4,4'-bromphenyl)-(5-thiazolyl)-methan in DMF mit CuCN in die Titelverbindung überführt, Smp. 54-56°.

Das Ausgangsmaterial wird wie folgt hergestellt:

### Beispiel 13 (a): Bis-(4,4'-bromphenyl)-(5-thiazolyl)-methanol

Analog Beispiel 3 wird ausgehend von 0,68 ml Diisopropylamin, 2 ml 1,6 M n-Butyllithium-Lösung, 0,63 g 2-Trimethylsilylthiazol, 1,36 g 4,4'-Dibrombenzophenon in THF bei -75° und gleicher Aufarbeitung die Titelverbindung hergestellt. Diese wird durch Säulenchromatographie (SiO₂, 0,1 bar, Hexan/Ethylacetat) gereinigt; ¹H-NMR (CDCl₃): δ (ppm) = 4,14 (br,1H), 7,25 und 7,48 (m,8H), 7,36 (d,1H), 7,85 (d,1H).

### Beispiel 13 (b): Bis-(4,4'-bromphenyl)-(5-thiazolyl)-methan

Analog Beispiel 4 wird Bis-(4,4'-bromphenyl)-(5-thiazolyl)-methanol in Eisessig mit Zinn(II)chlorid und 36,5 % Salzsäure in die Titelverbindung überführt.

### Beispiel 14: 4-[α-(4-Cyanophenyl)-α-fluor-5-isothiazolylmethyl]-benzonitril

4-[α-(4-Cyanophenyl)-5-isothiazolylmethyl]-benzonitril (Beispiel 2) wird durch Behandlung mit Lithiumdiisopropylamid in THF bei -78° in das Carbanion überführt und dann mit N-Fluor-3,3-dimethyl-2,3-dihydro-1,2-benzothiazol-1,1-dioxid umgesetzt. Man lässt auf Raumtemperatur erwärmen und arbeitet das Reaktionsgemisch wässrig auf, wobei man die Titelverbindung erhält.

### Beispiel 15: 6-Cyano-1-(5-isothiazolyl)-1,2,3,4-tetrahydronaphthalin

Analog Beispiel 4 wird 6-Cyano-1-hydroxy-1-(5-isothiazolyl)-1,2,3,4-tetrahydronaphthalin (Beispiel 7) in Eisessig mit Zinn(II)chlorid und 36,5 % Salzsäure in die Titelverbindung überführt.

### Beispiel 16: 6-Cyano-1-fluor-1-(5-isothiazolyl)-1,2,3,4-tetrahydronaphthalin

6-Cyano-1-(5-isothiazolyl)-1,2,3,4-tetrahydronaphthalin (Beispiel 15) wird durch Behandlung mit Lithiumdiisopropylamid in THF bei -78° in das Carbanion überführt und dann mit N-Fluor-3,3-dimethyl-2,3-dihydro-1,2-benzothiazol-1,1-dioxid umgesetzt. Man lässt auf Raumtemperatur erwärmen und arbeitet das Reaktionsgemisch wässrig auf, wobei man die Titelverbindung erhält.

### Beispiel 17:

Es werden 10000 Tabletten hergestellt, die je 5 mg Wirkstoff, z.B. eine der in den Beispielen 1-16 hergestellten Verbindungen, enthalten:

| Zusammensetzung: | |
|---|---|
| Wirkstoff | 50,00 g |
| Milchzucker | 2535,00 g |
| Maisstärke | 125,00 g |
| Polyethylenglykol 6.000 | 150,00 g |
| Magnesiumstearat | 40,00 g |
| Gereinigtes Wasser | quantum satis |

Verfahren: Alle pulverigen Bestandteile werden durch ein Sieb mit einer Maschenweite von 0,6 mm gesiebt. Dann wird der Wirkstoff, der Milchzucker, das Magnesiumstearat und die Hälfte der Stärke in einem geeigneten Mischer vermischt. Die andere Hälfte der Stärke wird in 65 ml Wasser suspendiert und die entstandene Suspension zu einer siedenden Lösung des Polyethylenglykols in 260 ml Wasser gegeben. Die gebildete Paste wird dem Pulvergemisch zugesetzt und gegebenenfalls unter Zugabe von mehr Wasser granuliert. Das Granulat wird über Nacht bei 35°C getrocknet, durch ein Sieb mit 1,2 mm Maschenweite getrieben und zu Tabletten, welche eine Bruchrille aufweisen, gepresst.

### Beispiel 18:

Es werden 1000 Kapseln hergestellt, die je 10 mg Wirkstoff, z.B. eine der in den Beispielen 1-16 hergestellten Verbindungen, enthalten:

| Zusammensetzung: | |
|---|---|
| Wirkstoff | 10,00 g |
| Milchzucker | 207,00 g |
| Modifizierte Stärke | 80,00 g |
| Magnesiumstearat | 3,00 g |

Verfahren: Alle pulverigen Bestandteile werden durch ein Sieb mit einer Maschenweite von 0,6 mm gesiebt. Dann wird der Wirkstoff in einem geeigneten Mischer zuerst mit dem Magnesiumstearat und dann mit dem Milchzucker und der Stärke bis zur Homogenität vermischt. Hartgelatinekapseln Nr. 2 werden mit je 300 mg der erhaltenen Mischung unter Verwendung einer Kapselfüllmaschine gefüllt.

## Patentansprüche (Patentansprüche für folgende(n) Vertragsstaat(en): AT, BE, CH, LI, DE, DK, FR, GB, IT, LU, NL, SE)

1. Verbindungen der Formel I, 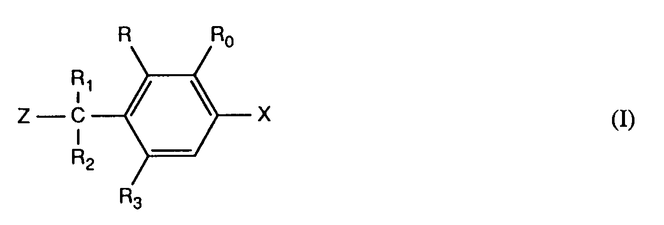 worin Z einen fünfgliedrigen, Stickstoff enthaltenden heteroaromatischen Ring ausgewählt aus der Gruppe 5-Isothiazolyl, 5-Thiazolyl, 5-Isoxazolyl, 5-Oxazolyl, 5-(1,2,3-Thiadiazolyl), 5-(1,2,3-Oxadiazolyl), 3-(1,2,5-Thiadiazolyl), 3-(1,2,5-Oxadiazolyl), 4-Isothiazolyl, 4-Isoxazolyl, 4-(1,2,3-Thiadiazolyl), 4-(1,2,3-Oxadiazolyl), 2-(1,3,4-Thiadiazolyl), 2-(1,3,4-Oxadiazolyl), 5-(1,2,4-Thiadiazolyl) und 5-(1,2,4-Oxadiazolyl) bedeutet; R, R₀ und R₃ Wasserstoff bedeuten; R₁ Wasserstoff, Methyl, Hydroxy oder Fluor bedeutet; R₂ für Cyanophenyl steht; oder R₁ und R₂ zusammen Methyliden bedeuten; oder R₂ und R₃ zusammen -(CH₂)₃- bedeuten; oder R₁ und R₂ und R₃ zusammen für eine Gruppe =CH-(CH₂)₂- stehen, wobei die Einfachbindung am Benzolring angeknüpft ist; und X für Cyano steht; und Salze davon.

2. Verbindungen der Formel I gemäss Anspruch 1, worin Z 5-Isothiazolyl oder 5-Thiazolyl bedeutet; R, R₀ und R₃ Wasserstoff bedeuten; R₁ Wasserstoff, Methyl, Hydroxy oder Fluor bedeutet; R₂ für Cyanophenyl steht; oder R₁ und R₂ zusammen Methyliden bedeuten; oder R₂ und R₃ zusammen -(CH₂)₃- bedeuten; oder R₁ und R₂ und R₃ zusammen für eine Gruppe =CH-(CH₂)₂- stehen, wobei die Einfachbindung am Benzolring angeknüpft ist; und X für Cyano steht; und Salze davon.

3. Verbindungen der Formel I gemäss Anspruch 1, worin Z 5-Isothiazolyl oder 5-Thiazolyl bedeutet; R, R₀ und R₃ Wasserstoff bedeuten; R₁ Wasserstoff, Hydroxy oder Fluor bedeutet; R₂ für Cyanophenyl steht; oder R₁ und R₂ zusammen Methyliden bedeuten; oder R₂ und R₃ zusammen -(CH₂)₃- bedeuten; oder R₁ und R₂ und R₃ zusammen für eine Gruppe =CH-(CH₂)₂- stehen, wobei die Einfachbindung am Benzolring angeknüpft ist; und X für Cyano steht; und Salze davon.

4. 4-[α-(4-Cyanophenyl)-5-isothiazolylmethyl]-benzonitril gemäss Anspruch 1, oder ein pharmazeutisch verwendbares Salz davon.

5. 1-(4-Cyanophenyl)-1-(5-thiazolyl)-ethylen gemäss Anspruch 1, oder ein pharmazeutisch verwendbares Salz davon.

6. 6-Cyano-1-(5-isothiazolyl)-3,4-dihydronaphthalin gemäss Anspruch 1, oder ein pharmazeutisch verwendbares Salz davon.

7. Pharmazeutische Präparate enthaltend eine Verbindung gemäss einem der Ansprüche 1 bis 6 und mindestens ein pharmazeutisch verwendbares Trägermaterial.

8. Eine Verbindung gemäss einem der Ansprüche 1 bis 6 zur Anwendung in einem Verfahren zur therapeutischen Behandlung des tierischen oder menschlichen Körpers.

9. Eine Verbindung gemäss einem der Ansprüche 1 bis 6 zur Verwendung bei der Behandlung von Krankheiten, die auf eine Hemmung des Enzyms Aromatase ansprechen.

10. Verwendung einer Verbindung gemäss einem der Ansprüche 1 bis 6 zur Herstellung pharmazeutischer Präparate.

11. Verwendung einer Verbindung gemäss einem der Ansprüche 1 bis 6 zur Herstellung pharmazeutischer Präparate für die Behandlung von Krankheiten, die auf eine Hemmung des Enzyms Aromatase ansprechen.

12. Verfahren zur Herstellung einer Verbindung der Formel I gemäss Anspruch 1, dadurch gekennzeichnet, dass man
(a) zur Herstellung einer Verbindung der Formel I, worin R₁ Hydroxy bedeutet, eine Verbindung der Formel II, 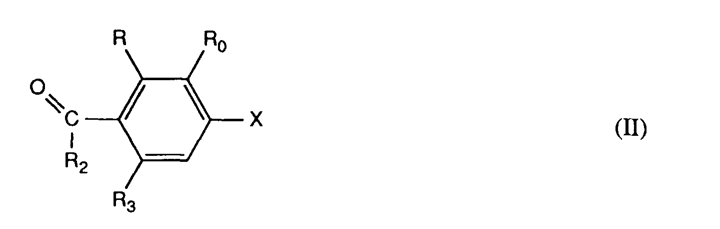 worin X, R, R₀, R₂ und R₃ wie unter Formel I definiert sind, mit einer Verbindung der Formel III,
Z-R₄ (III)
worin R₄ für Wasserstoff oder eine an einem Ringkohlenstoff sitzende Schutzgruppe oder Abgangsgruppe steht und Z wie unter Formel I definiert ist, umsetzt, oder
(b) zur Herstellung einer Verbindung der Formel I, worin R₁ und R₂ zusammen Methyliden bedeuten, eine Verbindung der Formel IV, 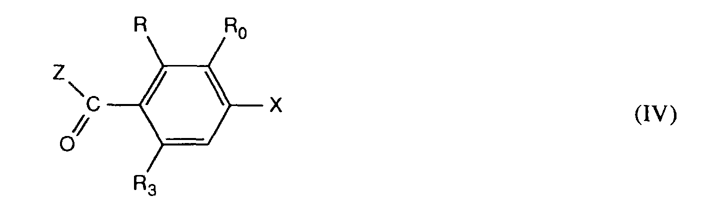 worin Z, R, R₀, R₃ und X wie unter Formel I definiert sind, mit einer Verbindung der Formel V,
Alk = W₁ (V)
worin Alk Methyliden und W₁ eine Phosphoranylidengruppe bedeutet, umsetzt, oder
(c) in einer Verbindung der Formel VI, 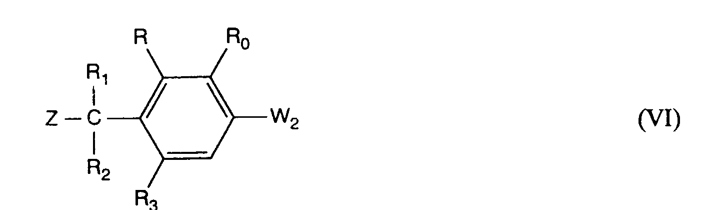 worin Z, R, R₀, R₁, R₂ und R₃ wie unter Formel I definiert sind und W₂ eine in den Rest X überführbare Gruppe bedeutet, W₂ in den Rest X überführt, wobei gegebenenfalls ein mit W₂ identischer Substituent innerhalb der Gruppe R₂ gleichzeitig umgewandelt wird, oder
(d) zur Herstellung einer Verbindung der Formel I, worin R₁ Wasserstoff bedeutet, eine Verbindung der Formel I, worin R₁ Hydroxy bedeutet, reduziert, oder
(e) zur Herstellung einer Verbindung der Formel I, worin R₁ und R₂ und R₃ zusammen für eine Gruppe =CH-(CH₂)₂- stehen, aus einer Verbindung der Formel I, worin R₂ und R₃ zusammen -(CH₂)₃- bedeuten und R₁ für Hydroxy steht, Wasser eliminiert, oder
(f) zur Herstellung einer Verbindung der Formel I, worin R₁ Methyl bedeutet, eine Verbindung der Formel I, worin R₁ Wasserstoff bedeutet, mit Methanol, oder einem reaktiven funktionellen Derivat davon, kondensiert, und/oder
eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I in ein Salz umwandelt, und/oder ein erhaltenes Gemisch von isomeren Verbindungen der Formel I in die einzelnen Isomeren auftrennt.

13. Bis-(4,4'-bromphenyl)-(5-isothiazolyl)-methan, oder ein pharmazeutisch verwendbares Salz davon.

## Patentansprüche (Patentansprüche für folgende(n) Vertragsstaat(en): ES, GR)

1. Verfahren zur Herstellung von Verbindungen der Formel I, 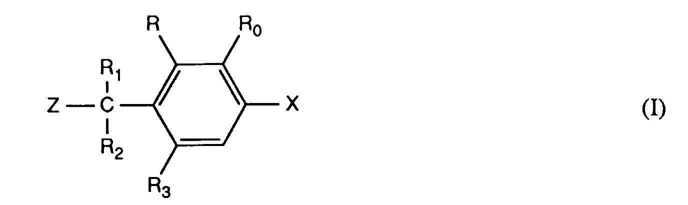 worin Z einen fünfgliedrigen, Stickstoff enthaltenden heteroaromatischen Ring ausgewählt aus der Gruppe 5-Isothiazolyl, 5-Thiazolyl, 5-Isoxazolyl, 5-Oxazolyl, 5-(1,2,3-Thiadiazolyl), 5-(1,2,3-Oxadiazolyl), 3-(1,2,5-Thiadiazolyl), 3-(1,2,5-Oxadiazolyl), 4-Isothiazolyl, 4-Isoxazolyl, 4-(1,2,3-Thiadiazolyl), 4-(1,2,3-Oxadiazolyl), 2-(1,3,4-Thiadiazolyl), 2-(1,3,4-Oxadiazolyl), 5-(1,2,4-Thiadiazolyl) und 5-(1,2,4-Oxadiazolyl) bedeutet; R, R₀ und R₃ Wasserstoff bedeuten; R₁ Wasserstoff, Methyl, Hydroxy oder Fluor bedeutet; R₂ für Cyanophenyl steht; oder R₁ und R₂ zusammen Methyliden bedeuten; oder R₂ und R₃ zusammen -(CH₂)₃- bedeuten; oder R₁ und R₂ und R₃ zusammen für eine Gruppe =CH-(CH₂)₂- stehen, wobei die Einfachbindung am Benzolring angeknüpft ist; und X für Cyano steht; und Salzen davon, dadurch gekennzeichnet, dass man
(a) zur Herstellung einer Verbindung der Formel I, worin R₁ Hydroxy bedeutet, eine Verbindung der Formel II, 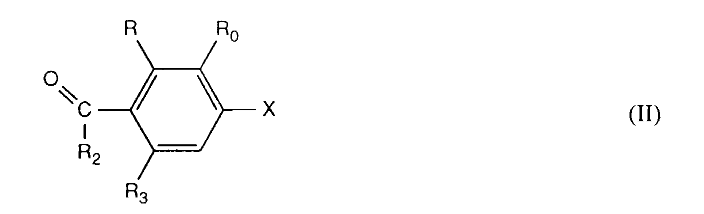 worin X, R, R₀, R₂ und R₃ wie unter Formel I definiert sind, mit einer Verbindung der Formel III,
Z-R₄ (III)
worin R₄ für Wasserstoff oder eine an einem Ringkohlenstoff sitzende Schutzgruppe oder Abgangsgruppe steht und Z wie unter Formel I definiert ist, umsetzt, oder
(b) zur Herstellung einer Verbindung der Formel I, worin R₁ und R₂ zusammen Methyliden bedeuten, eine Verbindung der Formel IV, 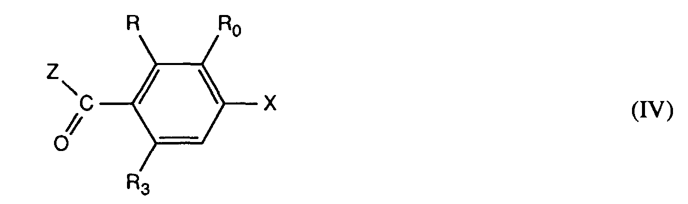 worin Z, R, R₀, R₃ und X wie unter Formel I definiert sind, mit einer Verbindung der Formel V,
Alk = W₁ (V)
worin Alk Methyliden und W₁ eine Phosphoranylidengruppe bedeutet, umsetzt, oder
(c) in einer Verbindung der Formel VI, 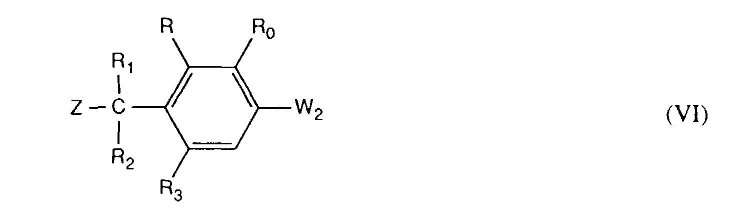 worin Z, R, R₀, R₁, R₂ und R₃ wie unter Formel I definiert sind und W₂ eine in den Rest X überführbare Gruppe bedeutet, W₂ in den Rest X überführt, wobei gegebenenfalls ein mit W₂ identischer Substituent innerhalb der Gruppe R₂ gleichzeitig umgewandelt wird, oder
(d) zur Herstellung einer Verbindung der Formel I, worin R₁ Wasserstoff bedeutet, eine Verbindung der Formel I, worin R₁ Hydroxy bedeutet, reduziert, oder
(e) zur Herstellung einer Verbindung der Formel I, worin R₁ und R₂ und R₃ zusammen für eine Gruppe =CH-(CH₂)₂- stehen, aus einer Verbindung der Formel I, worin R₂ und R₃ zusammen -(CH₂)₃- bedeuten und R₁ für Hydroxy steht, Wasser eliminiert, oder
(f) zur Herstellung einer Verbindung der Formel I, worin R₁ Methyl bedeutet, eine Verbindung der Formel I, worin R₁ Wasserstoff bedeutet, mit Methanol, oder einem reaktiven funktionellen Derivat davon, kondensiert, und/oder
eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I in ein Salz umwandelt, und/oder ein erhaltenes Gemisch von isomeren Verbindungen der Formel I in die einzelnen Isomeren auftrennt.

2. Verfahren gemäss Anspruch 1 zur Herstellung von Verbindungen der Formel I, worin Z 5-Isothiazolyl oder 5-Thiazolyl bedeutet; R, R₀ und R₃ Wasserstoff bedeuten; R₁ Wasserstoff, Methyl, Hydroxy oder Fluor bedeutet; R₂ für Cyanophenyl steht; oder R₁ und R₂ zusammen Methyliden bedeuten; oder R₂ und R₃ zusammen -(CH₂)₃- bedeuten; oder R₁ und R₂ und R₃ zusammen für eine Gruppe =CH-(CH₂)₂- stehen, wobei die Einfachbindung am Benzolring angeknüpft ist; und X für Cyano steht; und Salzen davon, dadurch gekennzeichnet, dass man entsprechend substituierte Ausgangsmaterialien verwendet.

3. Verfahren gemäss Anspruch 1 zur Herstellung von Verbindungen der Formel I, worin Z 5-Isothiazolyl oder 5-Thiazolyl bedeutet; R, R₀ und R₃ Wasserstoff bedeuten; R₁ Wasserstoff, Hydroxy oder Fluor bedeutet; R₂ für Cyanophenyl steht; oder R₁ und R₂ zusammen Methyliden bedeuten; oder R₂ und R₃ zusammen -(CH₂)₃- bedeuten; oder R₁ und R₂ und R₃ zusammen für eine Gruppe =CH-(CH₂)₂- stehen, wobei die Einfachbindung am Benzolring angeknüpft ist; und X für Cyano steht; und Salzen davon, dadurch gekennzeichnet, dass man entsprechend substituierte Ausgangsmaterialien verwendet.

4. Verfahren gemäss Anspruch 1 zur Herstellung von 4-[α-(4-Cyanophenyl)-5-isothiazolylmethyl]-benzonitril der Formel I, oder einem pharmazeutisch verwendbaren Salz davon, dadurch gekennzeichnet, dass man entsprechend substituierte Ausgangsmaterialien verwendet.

5. Verfahren gemäss Anspruch 1 zur Herstellung von 1-(4-Cyanophenyl)-1-(5-thiazolyl)-ethylen der Formel I, oder einem pharmazeutisch verwendbaren Salz davon, dadurch gekennzeichnet, dass man entsprechend substituierte Ausgangsmaterialien verwendet.

6. Verfahren gemäss Anspruch 1 zur Herstellung von 6-Cyano-1-(5-isothiazolyl)-3,4-dihydronaphthalin der Formel I, oder einem pharmazeutisch verwendbaren Salz davon, dadurch gekennzeichnet, dass man entsprechend substituierte Ausgangsmaterialien verwendet.

7. Verfahren zur Herstellung eines pharmazeutischen Präparates zur Behandlung Oestrogen-abhängiger Erkrankungen, dadurch gekennzeichnet, dass man eine gemäss einem der in einem der Ansprüche 1 bis 6 genannten Verfahren hergestellte Verbindung der Formel I mit einem pharmazeutisch anwendbaren Trägermaterial mischt.

## Claims (Claims for the following Contracting State(s): AT, BE, CH, LI, DE, DK, FR, GB, IT, LU, NL, SE)

1. A compound of formula I 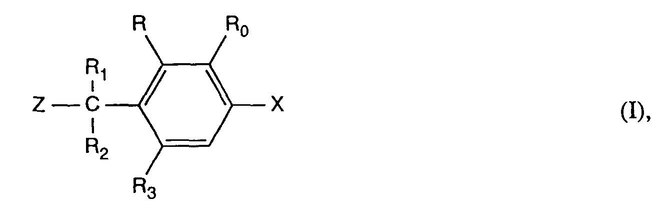 wherein Z is a five-membered nitrogen-containing heteroaromatic ring selected from the group 5-isothiazolyl, 5-thiazolyl, 5-isoxazolyl, 5-oxazolyl, 5-(1,2,3-thiadiazolyl), 5-(1,2,3-oxadiazolyl), 3-(1,2,5-thiadiazolyl), 3-(1,2,5-oxadiazolyl), 4-isothiazolyl, 4-isoxazolyl, 4-(1,2,3-thiadiazolyl), 4-(1,2,3-oxadiazolyl), 2-(1,3,4-thiadiazolyl), 2-(1,3,4-oxadiazolyl), 5-(1,2,4-thiadiazolyl) and 5-(1,2,4-oxadiazolyl); R, R₀ and R₃ are hydrogen; R₁ is hydrogen, methyl, hydroxy or fluorine; R₂ is cyanophenyl; or R₁ and R₂ together are methylidene; or R₂ and R₃ together are -(CH₂)₃-; or R₁ and R₂ and R₃ together are a group =CH-(CH₂)₂-, the single bond being linked to the benzene ring; and X is cyano; or a salt thereof.

2. A compound of formula I according to claim 1, wherein Z is 5-isothiazolyl or 5-thiazolyl; R, R₀ and R₃ are hydrogen; R₁ is hydrogen, methyl, hydroxy or fluorine; R₂ is cyanophenyl; or R₁ and R₂ together are methylidene; or R₂ and R₃ together are -(CH₂)₃-; or R₁ and R₂ and R₃ together are a group =CH-(CH₂)₂-, the single bond being linked to the benzene ring; and X is cyano; or a salt thereof.

3. A compound of formula I according to claim 1, wherein Z is 5-isothiazolyl or 5-thiazolyl; R, R₀ and R₃ are hydrogen; R₁ is hydrogen, hydroxy or fluorine; R₂ is cyanophenyl; or R₁ and R₂ together are methylidene; or R₂ and R₃ together are -(CH₂)₃-; or R₁ and R₂ and R₃ together are a group =CH-(CH₂)₂-, the single bond being linked to the benzene ring; and X is cyano; or a salt thereof.

4. 4-[α-(4-Cyanophenyl)-5-isothiazolylmethyl]-benzonitrile according to claim 1, or a pharmaceutically acceptable salt thereof.

5. 1-(4-Cyanophenyl)-1-(5-thiazolyl)-ethylene according to claim 1, or a pharmaceutically acceptable salt thereof.

6. 6-Cyano-1-(5-isothiazolyl)-3,4-dihydronaphthalene according to claim 1, or a pharmaceutically acceptable salt thereof.

7. A pharmaceutical composition comprising a compound according to any one of claims 1 to 6 and at least one pharmaceutically acceptable carrier.

8. A compound according to any one of claims 1 to 6 for use in a method for the therapeutic treatment of the animal or human body.

9. A compound according to any one of claims 1 to 6 for use in the treatment of disorders that respond to inhibition of the enzyme aromatase.

10. The use of a compound according to any one of claims 1 to 6 for the preparation of pharmaceutical compositions.

11. The use of a compound according to any one of claims 1 to 6 for the preparation of pharmaceutical compositions for the treatment of disorders that respond to inhibition of the enzyme aromatase.

12. A process for the preparation of a compound of formula I according to claim 1, which comprises
(a) for the preparation of a compound of formula I wherein R₁ is hydroxy, reacting a compound of formula II 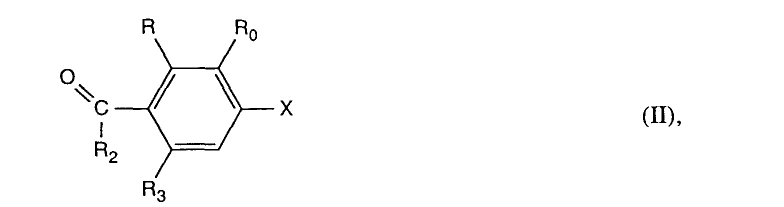 wherein X, R, R₀, R₂ and R₃ are as defined for formula I, with a compound of formula III
Z-R₄ (III)
wherein R₄ is hydrogen or a protecting group or leaving group attached to a ring carbon atom and Z is as defined for formula I, or
(b) for the preparation of a compound of formula I wherein R₁ and R₂ together are methylidene, reacting a compound of formula IV 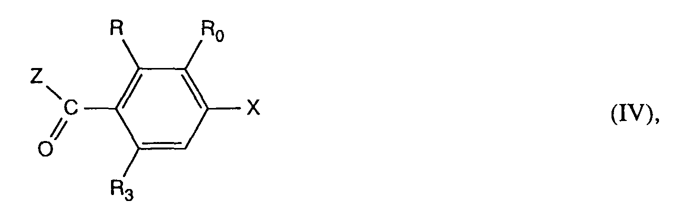 wherein Z, R, R₀, R₃ and X are as defined for formula I, with a compound of formula V
Alk = W₁ (V)
wherein Alk is methylidene and W₁ is a phosphoranylidene group, or
(c) in a compound of formula VI 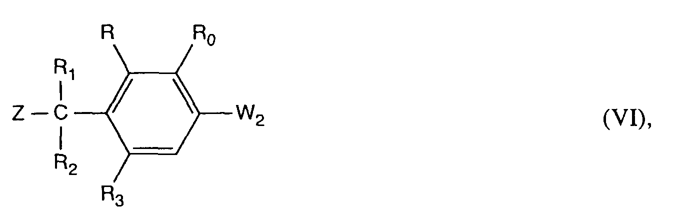 wherein Z, R, R₀, R₁, R₂ and R₃ are as defined for formula I and W₂ is a group that can be converted into the radical X, converting W₂ into the radical X, wherein a substituent identical to W₂ within the group R₂ may be converted simultaneously, or
(d) for the preparation of a compound of formula I wherein R₁ is hydrogen, reducing a compound of formula I wherein R₁ is hydroxy, or
(e) for the preparation of a compound of formula I wherein R₁ and R₂ and R₃ together are a group =CH-(CH₂)₂-, eliminating the elements of water from a compound of formula I wherein R₂ and R₃ together are -(CH₂)₃- and R₁ is hydroxy, or
(f) for the preparation of a compound of formula I wherein R₁ is methyl, condensing a compound of formula I wherein R₁ is hydrogen with methanol, or with a reactive functional derivative thereof, and/or
converting a resulting compound of formula I into a different compound of formula I, and/or converting a resulting salt into the free compound or into a different salt, and/or converting a resulting free compound of formula I into a salt, and/or separating a resulting mixture of isomeric compounds of formula I into the individual isomers.

13. Bis(4,4'-bromophenyl)-(5-isothiazolyl)methane, or a pharmaceutically acceptable salt thereof.

## Claims (Claims for the following Contracting State(s): ES, GR)

1. A process for the preparation of a compound of formula I 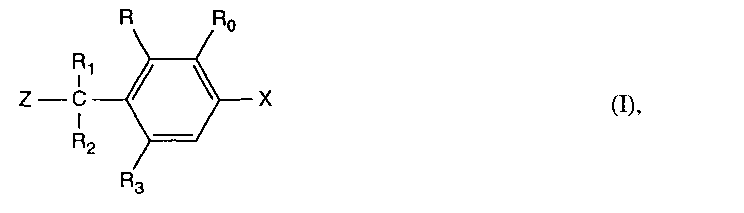 wherein Z is a five-membered nitrogen-containing heteroaromatic ring selected from the group 5-isothiazolyl, 5-thiazolyl, 5-isoxazolyl, 5-oxazolyl, 5-(1,2,3-thiadiazolyl), 5-(1,2,3-oxadiazolyl), 3-(1,2,5-thiadiazolyl), 3-(1,2,5-oxadiazolyl), 4-isothiazolyl, 4-isoxazolyl, 4-(1,2,3-thiadiazolyl), 4-(1,2,3-oxadiazolyl), 2-(1,3,4-thiadiazolyl), 2-(1,3,4-oxadiazolyl), 5-(1,2,4-thiadiazolyl) and 5-(1,2,4-oxadiazolyl); R, R₀ and R₃ are hydrogen; R₁ is hydrogen, methyl, hydroxy or fluorine; R₂ is cyanophenyl; or R₁ and R₂ together are methylidene; or R₂ and R₃ together are -(CH₂)₃-; or R₁ and R₂ and R₃ together are a group =CH-(CH₂)₂-, the single bond being linked to the benzene ring; and X is cyano; or a salt thereof, which process comprises:
(a) for the preparation of a compound of formula I wherein R₁ is hydroxy, reacting a compound of formula II 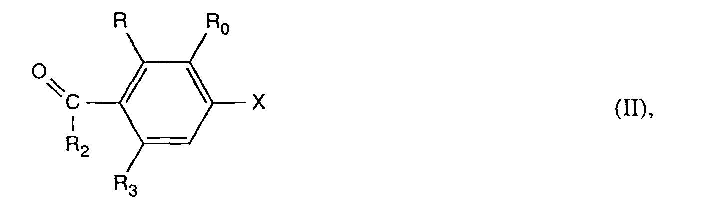 wherein X, R, R₀, R₂ and R₃ are as defined for formula I, with a compound of formula III
Z-R₄ (III),
wherein R₄ is hydrogen or a protecting group or leaving group attached to a ring carbon atom and Z is as defined for formula I, or
(b) for the preparation of a compound of formula I wherein R₁ and R₂ together are methylidene, reacting a compound of formula IV 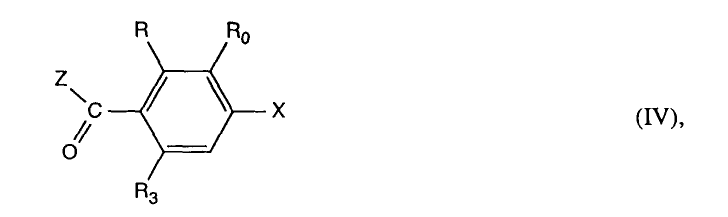 wherein Z, R, R₀, R₃ and X are as defined for formula I, with a compound of formula V
Alk = W₁ (V),
wherein Alk is methylidene and W₁ is a phosphoranylidene group, or
(c) in a compound of formula VI 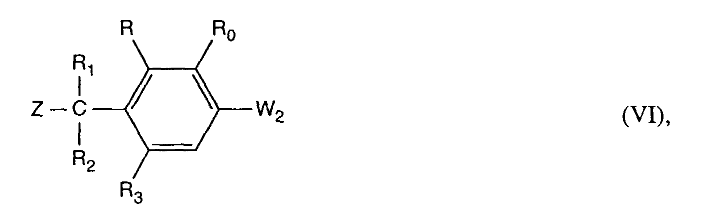 wherein Z, R, R₀, R₁, R₂ and R₃ are as defined for formula I and W₂ is a group that can be converted into the radical X, converting W₂ into the radical X, wherein a substituent identical to W₂ within the group R₂ may be converted simultaneously, or
(d) for the preparation of a compound of formula I wherein R₁ is hydrogen, reducing a compound of formula I wherein R₁ is hydroxy, or
(e) for the preparation of a compound of formula I wherein R₁ and R₂ and R₃ together are a group =CH-(CH₂)₂-, eliminating the elements of water from a compound of formula I wherein R₂ and R₃ together are -(CH₂)₃- and R₁ is hydroxy, or
(f) for the preparation of a compound of formula I wherein R₁ is methyl, condensing a compound of formula I wherein R₁ is hydrogen with methanol, or with a reactive functional derivative thereof, and/or
converting a resulting compound of formula I into a different compound of formula I, and/or converting a resulting salt into the free compound or into a different salt, and/or converting a resulting free compound of formula I into a salt, and/or separating a resulting mixture of isomeric compounds of formula I into the individual isomers.

2. A process according to claim 1 for the preparation of a compound of formula I wherein Z is 5-isothiazolyl or 5-thiazolyl; R, R₀ and R₃ are hydrogen; R₁ is hydrogen, methyl, hydroxy or fluorine; R₂ is cyanophenyl; or R₁ and R₂ together are methylidene; or R₂ and R₃ together are -(CH₂)₃-; or R₁ and R₂ and R₃ together are a group =CH-(CH₂)₂-, the single bond being linked to the benzene ring; and X is cyano; or a salt thereof, which process comprises the use of suitably substituted starting materials.

3. A process according to claim 1 for the preparation of a compound of formula I wherein Z is 5-isothiazolyl or 5-thiazolyl; R, R₀ and R₃ are hydrogen; R₁ is hydrogen, hydroxy or fluorine; R₂ is cyanophenyl; or R₁ and R₂ together are methylidene; or R₂ and R₃ together are -(CH₂)₃-; or R₁ and R₂ and R₃ together are a group =CH-(CH₂)₂-, the single bond being linked to the benzene ring; and X is cyano; or a salt thereof, which process comprises the use of suitably substituted starting materials.

4. A process according to claim 1 for the preparation of 4-[α-(4-cyanophenyl)-5-isothiazolylmethyl]-benzonitrile of formula I, or a pharmaceutically acceptable salt thereof, which process comprises the use of suitably substituted starting materials.

5. A process according to claim 1 for the preparation of 1-(4-cyanophenyl)-1-(5-thiazolyl)-ethylene of formula I, or a pharmaceutically acceptable salt thereof, which process comprises the use of suitably substituted starting materials.

6. A process according to claim 1 for the preparation of 6-cyano-1-(5-isothiazolyl)-3,4-dihydronaphthalene of formula I, or a pharmaceutically acceptable salt thereof, which process comprises the use of suitably substituted starting materials.

7. A process for the preparation of a pharmaceutical composition for the treatment of oestrogen-dependent disorders, which process comprises mixing a compound of formula I prepared by one of the processes mentioned in any one of claims 1 to 6 with a pharmaceutically acceptable carrier.

## Revendications (Revendications pour l'(les) Etat(s) contractant(s) suivant(s): AT, BE, CH, LI, DE, DK, FR, GB, IT, LU, NL, SE)

1. Composés de formule I, 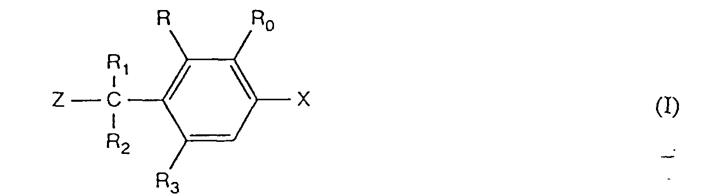 où z représente un cycle hétéroaromatique comprenant de l'azote à cinq maillons, choisi dans le groupe des 5-isothiazolyle, 5-thiazolyle, 5-isoxazolyle, 5-oxazolyle, 5-(1,2,3-thiadiazolyle), 5-(1,2,3-oxadiazolyle), 3-(1,2,5-thiadiazolyle), 3-(1,2,5-oxadiazolyle), 4-isothiazolyle, 4-isoxazolyle, 4-(1,2,3-thiadiazolyle), 4-(1,2,3-oxadiazolyle), 2-(1,3,4-thiadiazolyle), 2-(1,3,4-oxadiazolyle), 5-(1,2,4-thiadiazolyle) et 5-(1,2,4-oxadiazolyle) ; R, R₀ et R₃ représentent l'hydrogène ; R₁ représente un hydrogène, un méthyle, un hydroxy ou fluor ; R₂ représente un cyanophényle ; ou R₁ et R₂ représentent ensemble un méthylidène ; ou R₂ et R₃ représentent ensemble -(CH₂)₃- ; ou R₁ et R₂ représentent ensemble un groupe =CH-(CH₂)₂-, où la liaison simple est fixée sur le cycle benzénique ; et X représente un cyano ;
et leurs sels.

2. Composés de formule I selon la revendication 1, où Z représente un 5-isothiazolyle ou 5-thiazolyle ; R, R₀ et R₃ représentent l'hydrogène ; R₁ représente un hydrogène, méthyle, hydroxy ou fluor ; R₂ représente un cyanophényle ; ou R₁ et R₂ représentent ensemble un méthylidène ; ou R₂ et R₃ représentent ensemble -(CH₂)₃- ; ou R₁ et R₂ et R₃ représentent ensemble un groupe =CH- (CH₂)₂-, où la liaison simple est fixée sur le cycle benzénique ; et X représente un cyano ; et leurs sels.

3. Composés de formule I selon la revendication 1, où Z représente un 5-isothiazolyle ou un 5-thiazolyle ; R, R₀ et R₃ représentent un hydrogène ; R₁ représente un hydrogène, hydroxy ou fluor ; R₂ représente un cyanophényle ; ou R₁ et R₂ forment ensemble un méthylidène ; ou R₂ et R₃ forment ensemble -(CH₂)₃- ; ou R₁ et R₂ et R₃ représentent ensemble un groupe =CH (CH₂)₂- où la liaison simple est fixée sur le cycle benzénique ; et X représente un cyano ; et leurs sels.

4. 4,4-[α-(4-cyanophényl)-5-isothiazolylméthyl]benzonitrile selon la revendication 1, ou un de ses sels pharmaceutiquement utilisables.

5. 1-(4-cyanophényl)-1-(5-thiazolyl)-éthylène selon la revendication 1, ou un de ses sels pharmaceutiquement utilisables.

6. 6-cyano-1-(5-isothiazolyl)-3,4-dihydronaphtalène selon la revendication 1, ou un de ses sels pharmaceutiquement utilisables.

7. Préparations pharmaceutiques contenant un composé selon l'une des revendications 1 à 6 ou au moins un matériau véhicule pharmaceutiquement utilisable.

8. Composé selon l'une des revendications 1 à 6, pour l'utilisation dans un procédé de traitement thérapeutique du corps animal ou humain.

9. Composé selon l'une des revendications 1 à 6 pour le traitement de maladies qui répondent à une inhibition de l'enzyme aromatase.

10. Utilisation d'un composé selon l'une des revendications 1 à 6, pour produire des préparations pharmaceutiques.

11. Utilisation d'un composé selon l'une des revendications 1 à 6 pour produire des préparations pharmaceutiques pour le traitement de maladies qui répondent à une inhibition de l'enzyme aromatase.

12. Procédé de préparation d'un composé de formule I selon la revendication 1, caractérisé en ce qu'on
(a) fait réagir, pour produire un composé de formule I, où R₁ représente un hydroxy, un composé de formule II, 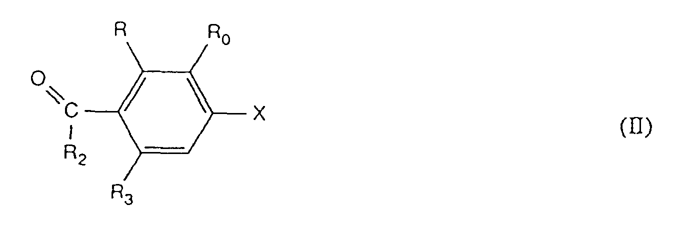 où X, R, R₀, R₂ et R₃ sont tels que définis à la formule I, avec un composé de formule III,
Z-R₄ (III)
où R₄ représente un hydrogène ou un groupe protecteur fixé sur un atome de carbone du cycle ou un groupe éliminable et Z est tel que défini pour la formule I, ou
(b) pour préparer un composé de formule I, où R₁ et R₂ représentent ensemble un méthylidène, on fait réaair un composé de formule IV, 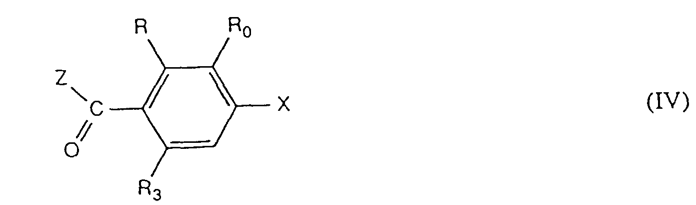 où Z, R, R₀, R₃ et X sont tels que définis à la formule I, avec un composé de formule V
Alk = W₁ (V)
où Alk représente un méthylidène et W₁ un groupe phosphoranylidène, ou
(c) dans un composé de formule VI, 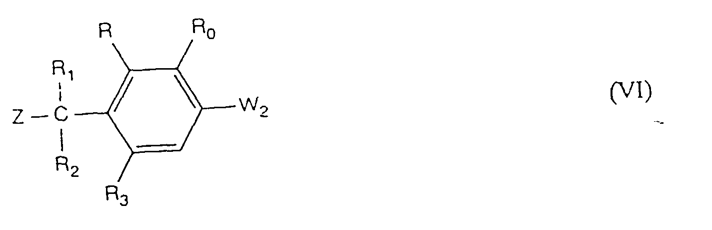 où Z, R, R₀, R₁, R₂ et R₃ sont tels que définis à la formule I et W₂ est un groupe transformable en le reste X, on transforme W₂ en le reste X, où on transforme le cas échéant un substituant identique à W₂ dans le groupe R₂, ou
(d) pour préparer un composé de formule I, où R₁ représente un hydrogène, on réduit un composé de formule I, où R₁ représente un hydroxy, ou
(e) pour produire un composé de formule I, où R₁ et R₂ et R₃ représentent ensemble un groupe =CH-(CH₂)₂-, on élimine l'eau d'un composé de formule I, où R₂ et R₃ forment ensemble -(CH₂)₃- et R₁ est un hydroxy, ou
(f) pour préparer un composé de formule I, où R₁ représente un méthyle, on condense un composé de formule I, où R₁ représente un hydrogène, avec du méthanol, ou un de ses dérivés fonctionnels réactifs, et/ou
on transforme un composé obtenu de formule I en un autre composé de formule I, et/ou on transforme un sel obtenu en le composé libre ou en un autre sel, et/ou on transforme un composé libre obtenu de formule I en un sel, et/ou on fractionne un mélange obtenu de composés isomères de formule I en les isomères individuels.

13. bis-(4,4'-bromophényl)-(5-isothiazolyl)-méthane ,ou un de ses sels pharmaceutiquement utilisables.

## Revendications (Revendications pour l'(les) Etat(s) contractant(s) suivant(s): ES, GR)

1. Procédé de préparation de composés de formule I, 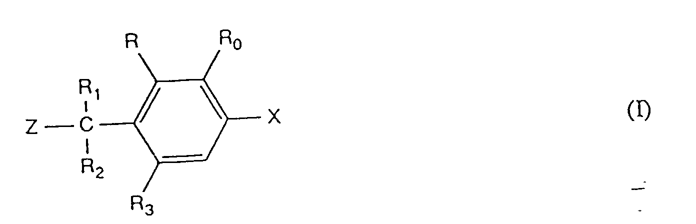 où Z représente un cycle hétéroaromatique comprenant de l'azote à cinq maillons, choisi dans le groupe des 5-isothiazolyle, 5-thiazolyle, 5-isoxazolyle, 5-oxazolyle, 5-(1,2,3-thiadiazolyle), 5-(1,2,3-oxadiazolyle), 3-(1,2,5-thiadiazolyle), 3-(1,2,5-oxadiazolyle), 4-isothiazolyle, 4-isoxazolyle, 4-(1,2,3-thiadiazolyle), 4-(1,2,3-oxadiazolyle), 2-(1,3,4-thiadiazolyle), 2-(1,3,4-oxadiazolyle), 5-(1,2,4-thiadiazolyle) et 5-(1,2,4-oxadiazolyle); R, R₀ et R₃ représentent l'hydrogène ; R₁ représente un hydrogène, un méthyle, un hydroxy ou fluor ; R₂ représente un cyanophényle ou R₁ et R₂ représentent ensemble un méthylidène ; ou R₂ et R₃ représentent ensemble -(CH₂)₃- ; ou R₁ et R₂ représentent ensemble un groupe =CH-(CH₂)₂-, où la liaison simple est fixée sur le cycle benzénique ; et X représente un cyano ; et leurs sels,
caractérisé en ce qu'on
(a) fait réagir, pour produire un composé de formule I, où R₁ représente un hydroxy, un composé de formule II, 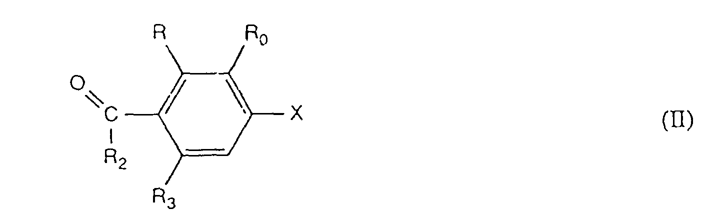 où X, R, R₀, R₂ et R₃ sont tels que définis à la formule I, avec un composé de formule III,
Z-R₄ (III)
où R₄ représente un hydrogène ou un groupe protecteur fixé sur un atome de carbone du cycle ou un groupe éliminable et Z est tel que défini pour la formule I, ou
(b) pour préparer un composé de formule I, où R₁ et R₂ représentent ensemble un méthylidène, on fait réagir un composé de formule IV, 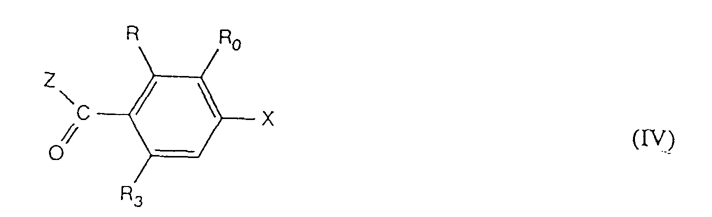 où Z, R, R₀, R₃ et X sont tels que définis à la formule I, avec un composé de formule V
Alk = W₁ (V)
où Alk représente un méthylidène et W₁ un groupe phosphoranylidène, ou
(c) dans un composé de formule VI, 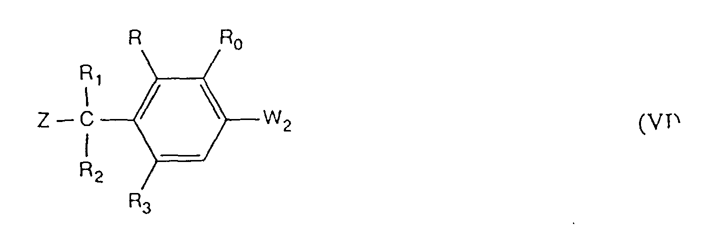 où Z, R, R₀, R₁, R₂ et R₃ sont tels que définis à la formule I et W₂ est un groupe transformable en le reste X, on transforme W₂ en le reste X, où on transforme le cas échéant un substituant identique à W₂ dans le groupe R₂, ou
(d) pour préparer un composé de formule I, où R₁ représente un hydrogène, on réduit un composé de formule I, où R₁ représente un hydroxy, ou
(e) pour produire un composé de formule I, où R₁ et R₂ et R₃ représentent ensemble un groupe =CH-(CH₂)₂-, on élimine l'eau d'un composé de formule I, où R₂ et R₃ forment ensemble -(CH₂)₃- et R₁ est un hydroxy, ou
(f) pour préparer un composé de formule I, où R₁ représente un méthyle, on condense un composé de formule I, où R₁ représente un hydrogène, avec du méthanol, ou un de ses dérivés fonctionnels réactifs, et/ou
on transforme un composé obtenu de formule I en un autre composé de formule I, et/ou on transforme un sel obtenu en le composé libre ou en un autre sel, et/ou on transforme un composé libre obtenu de formule I en un sel, et/ou on fractionne un mélange obtenu de composés isomères de formule I en les isomères individuels.

2. Procédé selon la revendication 1, de préparation de composés de formule I, où Z représente un 5-isothiazolyle ou 5-thiazolyle ; R, R₀ et R₃ représentent l'hydrogène ; R₁ représente un hydrogène, méthyle, hydroxy ou fluor ; R₂ représente un cyanophényle ; ou R₁ et R₂ représentent ensemble un méthylidène ou R₂ et R₃ représentent ensemble -(CH₂)₃- ; ou R₁ et R₂ et R₃ représentent ensemble un groupe =CH-(CH₂)₂-, où la liaison simple est fixée sur le cycle benzénique ; et X représente un cyano ; et leurs sels,
caractérisé en ce qu'on utilise les matériaux de départ substitués de manière appropriée.

3. Procédé selon la revendication 1, pour préparer des composés de formule I, où Z représente un 5-isothiazolyle ou un 5-thiazolyle ; R, R₀ et R₃ représentent un hydrogène ; R₁ représente un hydrogène, hydroxy ou fluor ; R₂ représente un cyanophényle ; ou R₁ et R₂ forment ensemble un méthylidène ; ou R₂ et R₃ forment ensemble -(CH₂)₃- ; ou R₁ et R₂ et R₃ représentent ensemble un groupe =CH(CH₂)₂- où la liaison simple est fixée sur le cycle benzénique ; et X représente un cyano ; et leurs sels,
caractérisé en ce qu'on utilise des matériaux de départ substitués de manière appropriée.

4. Procédé selon la revendication 1, pour préparer le 4-[α-(4-cyanophényl)-5-isothiazolylméthyl]-benzonitrile de formule I, ou un de ses sels pharmaceutiquement utilisables, caractérisé en ce qu'on utilise des matériaux de départ substitués de manière appropriée.

5. Procédé selon la revendication 1, pour préparer le 1-(4-cyanophényl)-1-(5-thiazolyl)-éthylène de formule I, ou un de ses sels pharmaceutiquement utilisables, caractérisé en ce qu'on utilise des matériaux de départ substitués de manière appropriée.

6. Procédé selon la revendication 1, pour préparer le 6-cyano-1-(5-isothiazolyl)-3,4-dihydronaphtalène de formule I, ou un de ses sels pharmaceutiquement utilisables, caractérisé en ce qu'on utilise des matériaux de départ substitués de manière appropriée.

7. Procédé de préparation d'une composition pharmaceutique pour traiter des maladies dépendantes des oestrogènes, caractérisé en ce qu'on mélange un composé préparé selon l'un des procédés décrits aux revendications 1 à 6 de formule I avec un matériau véhicule pharmaceutiquement utilisable.
